# EUROPEAN PATENT SPECIFICATION

(11) **EP 1 080 688 B1**
(45) Date of publication and mention of the grant of the patent: **24.11.2004**
(21) Application number: 99307714.8
(22) Date of filing: 30.09.1999
(51) Int. Cl.: A61B 5/145, A61B 5/15

(54) **Lancet**
Lanzette
Lancette

(30) Priority: 06.09.1999 PL 33527299
(43) Date of publication of application: 07.03.2001
(73) Proprietor: HTL Strefa Spolka z o.o., 95-035 Ozorkow (PL)
(72) Inventor: Rutynowski, Wlodzimierz, 00-770 Warszawa (PL); Wyszogrodzki, Wojciech, 03-289 Warszawa (PL)
(74) Representative: Fenlon, Christine Lesley

(56) References cited:
- DE-U- 8 900 203
- DE-U- 29 809 758

## Description

The subject of the present invention is a puncturing device in particular for puncturing skin of a patient in order to take a blood sample for diagnostic purposes.

A device for puncturing comprising a sleeve and a push element for actuating the device positioned at one end of the sleeve, is known from US patent specification No. 5,356,420. The opposite end of the sleeve is open. Inside the sleeve there is a slidably displacement piston, which at the end closer to the push element terminates in a head member, and which at the end closer to the open end of the sleeve terminates in a puncturing tip. Inside the sleeve, between the end face of the push element and the piston, is positioned a driving spring, and between the piston and the open end of the sleeve there is positioned a return spring. The piston has, at the outer circumference thereof, wings which rest on an internal projection of the sleeve.

US Patent specification No. 4,139,011 discloses a device for puncturing skin of a patient comprising a housing in the form of a sleeve closed at one end and which, at the other end, narrows and has a small opening. Inside the housing there is a slidably mounted rod, which at one end terminates in a slider positioned inside the housing. At the other end there is a rod comprising an elastic arm terminating in a holding tooth, which rests on the edge of the opening in the housing. Between the rod and the bottom of the sleeve is positioned a spring and, at the other end of the slider, a puncturing insert is provided.

EP-A-0 694 286 and DE 29809758 describe devices for actuating a lancet for puncturing the skin of a patient, which include a mechanism for pushing out and drawing in a lancet blade. The devices comprise a housing with an opening through which a lancet blade is pushed out. An actuating mechanism comprises a drive spring positioned inside the housing, which, when released, pushes out a lancet blade through the opening of the housing, the lancet blade puncturing a patient's skin and then being drawn back into the housing.

Finally, Japanese Patent specification No. 61-36607 describes a device for puncturing the skin, comprising a driving-triggering tubular unit and an attached container with a spring, for inserts terminated in a puncturing tip.

According to the present invention, there is provided a puncturing device, characterised in that it comprises a puncturing unit for receiving an exchangeable puncturing insert, a driving unit having a body connectable with the puncturing unit and comprising a ram positioned inside the body and a drive spring positioned around the ram between a first end of the ram pointing towards the position occupied by the puncturing insert, in use, and the body, the other end of the ram being retained in the body, and an actuating unit comprising a push element being positioned on the driving unit and having a projection therein directed towards said other end of the ram, wherein the projection is configured to release the ram by exerting a downwards force on its end when the push element is depressed.

Preferably, the puncturing unit comprises a housing having an opening at the bottom thereof and a first return spring is positioned within the housing. The puncturing insert may then be readily housed inside the housing of the puncturing unit.

The body of the driving unit preferably comprises an internal narrowing within which the ram is placed, the inner narrowing providing a base for the drive spring. The body of the driving unit may moreover, comprise a seat at which the aforesaid other end of the ram is positioned. The actuating unit itself may comprise a second return spring placed between the upper edge of the driving unit and the underside of the push element.

For a better understanding of the invention, and to show how the same may be carried into effect, reference will be made, by way of example only to the accompanying drawings, in which:-
Fig. 1 shows a longitudinal section through a puncturing device embodying this invention;
Fig. 2 shows a partly cut-away elevation of the puncturing device; and
Fig. 3 is a longitudinal section through the driving unit of the puncturing unit after releasing the ram.

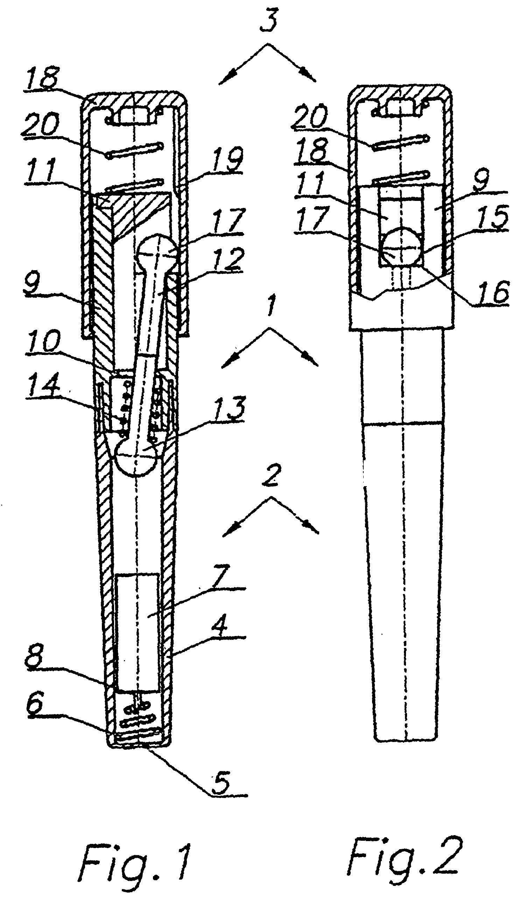

As shown in Figs. 1 and 2, a puncturing device embodying the invention comprises a driving unit 1, puncturing unit 2 and actuating unit 3. The puncturing unit 2 includes a housing 4, at the bottom of which is positioned an opening 5 within which a first return spring 6 is placed. In the housing 4 of the puncturing unit 2 is also positioned an exchangeable puncturing insert 7 which includes a puncturing needle 8 pointing towards the opening 5 positioned at the bottom of the housing 4. The driving unit 1, connected with puncturing unit 2, includes a body 9 whose free cross-section narrows at a neck 10 and which is open at one end. At the other end the body 9 is closed by a directing cap 11. In the neck 10 of the body 9 is positioned a ram 12. The ram 12 has one end 13 pointing towards the puncturing insert 7, and a drive spring 14 is positioned in the neck 10. The body 9 includes a seat 15 with an edge 16 supporting the other end 17 of the ram 12. The actuating unit 3 is placed on the driving unit 1 and includes a push element 18 with an internal projection 19 directed towards the other end 17 of the ram 12. A second return spring 20 is placed between upper edge of the driving unit 1 and the close end of the push element 18.

In order to use the puncturing device, the puncturing insert 7 is placed in the housing 4 of the puncturing unit 2, and then the puncturing unit 2 is connected to the driving unit 1. After placing a patient's finger to be punctured under opening 5 of the housing 4, the push element 18 of the actuating unit 3 is depressed, and, as the internal projection 19 of the push element 18 descends, it releases ram 12 of the driving unit 1 by exerting a downwards force on its end 17. This results in the extension of the drive spring 14 and the lower, first mentioned, end of the ram hits the puncturing insert 7. The puncturing needle 8 punctures the pationt's skin as it is driven through the opening 5 of the housing 4, then return spring 6 withdraws the puncturing insert 7 into the housing 4 of the puncturing unit 2. When the puncturing operation is complete, the puncturing unit 2 is disconnected from the driving unit 1, the skin sample in the puncturing insert 7 is retrieved and the puncturing insert 7 is thrown away.

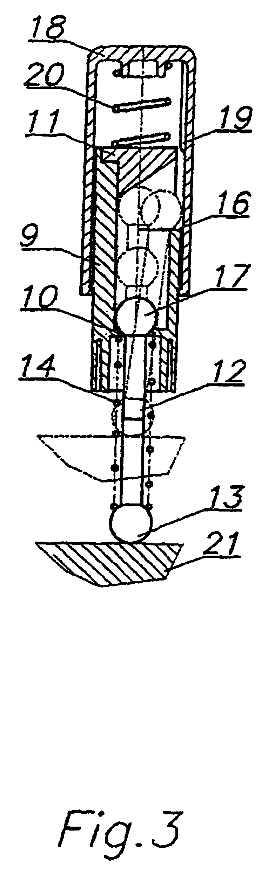

After collecting a sample, the driving unit is in the disposition shown in Fig. 3. To prepare the driving unit 1 for re-use, the first end 13 of ram 12 has to be placed against surface 21 and then the driving unit 1 has to be pushed towards said surface. In this way the other end 17 of the ram 12 slides in a directing cap 11 and assumes a stable position in seat 15 of the body 9 resting on edge 16, and the drive spring 14 is held in its compressed state. On placing another puncturing insert 7 in the housing 4 of the puncturing unit 2, and attaching the puncturing unit 2 to the driving unit 1, the puncturing device is ready to use again.

In a practical embodiment the puncturing device according to the invention may be provided with a number of different puncturing units, whose geometrical dimensions determine the depth of skin puncturing achieved therewith.

## Claims

1. A puncturing device, comprising a puncturing unit (2) for receiving an exchangeable puncturing insert (7), a driving unit (1) having a body (9) connectable with the puncturing unit (2) and comprising a ram (12) positioned inside the body (9), an actuating unit and a drive spring (14) positioned around the ram (12) between a first end (13) of the ram (12) pointing towards the position occupied by the puncturing insert (7), in use, and the body (9), the other end (17) of the ram (12) being retained in the body (9), and **characterised in that** the actuating unit (3) comprises a push element (18) positioned on the driving unit (1) and having a projection (19) therein directed towards said other end (17) of the ram (12), wherein the projection (19) is configured to release the ram (12) by exerting a downwards force on its end (17) when the push element (18) is depressed.

2. Device according to claim 1 wherein the puncturing unit (2) comprises a housing (4) having an opening (5) at the bottom thereof and a first return spring (6) is positioned within the said housing (4).

3. Device according claim 2 wherein the exchangeable puncturing insert (7) is received inside the housing (4) of the puncturing unit (2).

4. Device according to any of the preceding claims wherein the body (9) the driving unit (1) comprises an internal narrowing (10) within which the ram (12) is placed, and said inner narrowing (10) providing a base for the drive spring (14).

5. Device according to any of the preceding claims, wherein the body (9) of the driving unit (1) comprises a seat (15) at which said other end (17) of the ram (12) is positioned.

6. Device according to any of the preceding claims wherein the actuating unit (3) comprises a second return spring (20) positioned between the upper edge of the driving unit (1), and the underside of the push element (18).

## Patentansprüche

1. Punktionsvorrichtung, aufweisend eine Punktionseinheit (2) zum Aufnehmen eines austauschbaren Punktionseinschubes (7), eine Antriebseinheit (1) mit einem Körper (9), der mit der Punktionseinheit (2) verbindbar ist, und mit einem Stößel (12), der innerhalb des Körpers (9) positioniert ist, eine Betätigungseinheit und eine Antriebsfeder (14), die um den Stößel (12) herum zwischen einem ersten Ende (13) des Stößels (12), das gegen die vom Punktionseinschub (7) eingenommene Position bei der Verwendung zeigt, und dem Körper (9) positioniert ist, wobei das andere Ende (17) des Stößels (12) im Körper (9) gehalten wird, **dadurch gekennzeichnet, dass** die Betätigungseinheit (3) ein Schubelement (18) aufweist, das auf der Antriebseinheit (1) positioniert ist und einen Vorsprung (19) in sich aufweist, der gegen das andere Ende (17) des Stößels (12) gerichtet ist, wobei der Vorsprung (19) dafür konfiguriert ist, den Stößel (12) freizugeben, wenn eine Abwärtskraft auf sein Ende (17) beim Niederdrücken des Schubelements (18) ausgeübt wird.

2. Vorrichtung nach Anspruch 1, wobei die Punktionseinheit (2) ein Gehäuse (4) mit einer Öffnung (5) an seinem Boden aufweist und innerhalb des Gehäuses (4) eine erste Rückholfeder (6) positioniert ist.

3. Vorrichtung nach Anspruch 2, wobei der austauschbare Punktionseinschub (7) innerhalb des Gehäuses (4) der Punktionseinheit (2) aufgenommen ist

4. Vorrichtung nach einem der vorstehenden Ansprüche, wobei der Körper (9) der Antriebseinheit (1) eine interne Verengung (10) aufweist, innerhalb welcher der Stößel (12) angeordnet ist, und die innere Verengung (10) eine Basis für die Antriebsfeder (14) bildet.

5. Vorrichtung nach einem der vorstehenden Ansprüche, wobei der Körper (9) der Antreibseinheit (1) einen Sitz (15) aufweist, an dem das andere Ende (17) des Stößels (12) positioniert ist

6. Vorrichtung nach einem der vorstehenden Ansprüche, wobei die Betätigungseinheit (3) eine zweite Rückholfeder (20) aufweist, die zwischen der oberen Kante der Antriebseinheit (1) und der Unterseite des Schubelements (18) positioniert ist.

## Revendications

1. Dispositif de ponction comportant une unité de ponction (2) pour recevoir une pièce rapportée de ponction échangeable (7), une unité d'entraînement (1) ayant un corps (9) pouvant être connecté à l'unité de ponction (2), et comportant un piston (12) placé à l'intérieur du corps (9), une unité d'actionnement et un ressort d'entraînement (14) placés autour du piston (12) entre la première extrémité (13) du piston (12) dirigée vers la position occupée par la pièce rapportée de ponction (7) en utilisation et le corps (9), l'autre extrémité ( 17) du piston (12) étant retenue dans le corps (9), et
**caractérisé en ce que** l'unité d'actionnement (3) comporte un élément de poussée (18) placé sur l'unité d'entraînement (1), et ayant une saillie (19) à l'intérieur dirigée vers ladite autre extrémité (17) du piston (12), la saillie (19) étant configurée pour libérer le piston (12) en exerçant une force vers le bas sur son extrémité (17) lorsque l'élément de poussée (18) est enfoncé.

2. Dispositif selon la revendication 1, dans lequel l'unité de ponction (2) comporte un boîtier (4) ayant une ouverture (5) dans sa partie inférieure, et un premier ressort de rappel (6) est placé à l'intérieur dudit boîtier (4).

3. Dispositif selon la revendication 2, dans lequel la pièce rapportée de ponction échangeable (7) est reçue dans le boîtier (4) de l'unité de ponction (2).

4. Dispositif selon l'une quelconque des revendications précédentes, dans lequel le corps (9) de l'unité d'entraînement (1) comporte un rétrécissement interne (10) à l'intérieur duquel est placé le piston (12), et ledit rétrécissement interne (10) fournissant une base pour le ressort d'entraînement (14).

5. Dispositif selon l'une quelconque des revendications précédentes, dans lequel le corps (9) de l'unité d'entraînement (1) comporte un siège (15) dans lequel est placée ladite autre extrémité (17) du piston (12).

6. Dispositif selon l'une quelconque des revendications précédentes, dans lequel l'unité d'actionnement (3) comporte un second ressort de rappel (20) placé entre le bord supérieur de l'unité d'entraînement (1) et le côté inférieur de l'élément de poussée (18).
